(19) Europäisches Patentamt
European Patent Office
Office européen des brevets

(11) Veröffentlichungsnummer: **0 094 024**
**A1**

(12) # EUROPÄISCHE PATENTANMELDUNG

(21) Anmeldenummer: **83104387.2**

(51) Int. Cl.³: **C 01 B 33/28**, B 01 J 29/28

(22) Anmeldetag: **04.05.83**

(30) Priorität: **08.05.82 DE 3217324**

(43) Veröffentlichungstag der Anmeldung: **16.11.83**
**Patentblatt 83/46**

(84) Benannte Vertragsstaaten: **BE DE FR GB IT NL**

(71) Anmelder: **HOECHST AKTIENGESELLSCHAFT,**
**Postfach 80 03 20, D-6230 Frankfurt am Main 80 (DE)**

(72) Erfinder: **Baltes, Herbert, Dr., Johannesallee 24,**
**D-6230 Frankfurt am Main 80 (DE)**
Erfinder: **Litterer, Heinz, Dr., Albert-Schweitzer-Allee 39,**
**D-6200 Wiesbaden (DE)**
Erfinder: **Leupold, Ernst Ingo, Dr., Am Zäunefeld 15,**
**D-6392 Neu-Anspach (DE)**
Erfinder: **Wunder, Friedrich, Dr., Jahnstrasse 46,**
**D-6093 Flörsheim am Main (DE)**

(54) **Titanhaltige Zeolithe und Verfahren zu ihrer Herstellung sowie ihre Verwendung.**

(57) Die Erfindung betrifft titanhaltige Zeolithe, ein Verfahren zu deren Herstellung sowie ihre Verwendung. Zur Herstellung wird eine Mischung aus Silicium-, Aluminium-, Natrium-, Kalium-, Cholinverbindungen, Wasser sowie Titanverbindungen in spezifizierten Mengenverhältnissen hergestellt und in einem geschlossenen Gefäß erhitzt. Die Zeolithe finden Verwendung als Katalysatoren bei der Herstellung von $C_2$–$C_4$-Olefinen aus Methanol.

EP 0 094 024 A1

ACTORUM AG

Titanhaltige Zeolithe und Verfahren zu ihrer Herstellung sowie ihre Verwendung

Als Zeolithe bezeichnet man vor allem kristalline Aluminosilicate, bei denen durch eine dreidimensionale Verknüpfung von $SiO_4$- und $AlO_4$-Tetraedern regelmäßige Strukturen mit Hohlräumen und Poren entstehen. Im hydratisierten Zustand sind diese Poren und Hohlräume mit Wasser gefüllt. Dieses läßt sich ohne Beeinflussung der Kristallstruktur entfernen oder durch andere Moleküle ersetzen. Die negativen Ladungen der $AlO_4$-Tetraeder werden durch Kationen kompensiert. Diese können, wenn gewünscht, gegen andere Kationen ausgetauscht werden. Die geschilderten Eigenschaften ermöglichen die Verwendung der Zeolithe als Ionenaustauscher, Adsorbentien und Katalysatoren (D.W. Breck: Zeolite Molecular Sieves, 1974).

Zeolithe des X-, Y-, Mordenit, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom Pentasil-Typ (z.B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als Katalysatoren besteht großes Interesse an neuen Zeolithen mit spezifischen katalytischen Eigenschaften.

Beispielsweise erhält man sehr interessante Zeolithe, wenn man anstelle von Aluminium oder/und Silicium andere Elemente in das Zeolith-Gerüst einbaut. So wurden unter anderem Zeolithe der Pentasil-Reihe bekannt, die Bor (DE-OS 2 830 787), Eisen (DE-OS 2 831 611), Arsen (DE-AS 2 830 830), Antimon (DE-OS 2 830 787), Vanadin

(DE-OS 2 831 631), Chrom (DE-OS 2 831 630) oder Gallium (BE-PS 882 484) auf Tetraederplätzen enthalten. Auch wurden Titanosilicate (US-PS 3 329 481 und DE-OS 3 047 798) und Zirkonosilicate (US-PS 3 329 480) mit Zeolithstruktur bekannt.

Weiter wurden bereits beschrieben borhaltige Zeolithe, gallium- und/oder indiumhaltige Zeolithe, titanhaltige Zeolithe sowie zirkon- und/oder hafniumhaltige Zeolithe (deutsche Patentanmeldungen P 31 34 316.3, P 31 34 317.1, P 31 36 686.4, P 31 36 684.8, P 31 41 283.1, P 31 41 285.8).

Gegenstand der Erfindung sind titanhaltige Zeolithe, die dadurch gekennzeichnet sind, daß sie

a) außer Natrium, Kalium und Cholin die Elemente Silicium, Aluminium und Titan in folgendem Verhältnis enthalten, ausgedrückt in Molverhältnissen von Oxiden:

$$(SiO_2 + TiO_2) : (0,02 - 0,30)\ Al_2O_3$$

und

b) im Röntgenbeugungsdiagramm die in Tabelle 1 aufgeführten charakteristischen Signale aufweisen:

Tabelle 1

| Netzebenenabstände d/ Å | relative Intensität $I/I_o$ |
|---|---|
| 11,4 ± 0,3 | stark bis sehr stark |
| 9,2 ± 0,2 | schwach |
| 7,6 ± 0,2 | schwach bis mittel |
| 6,6 ± 0,1 | mittel bis stark |
| 5,7 ± 0,1 | schwach bis mittel |
| 5,35 ± 0,1 | schwach |
| 4,56 ± 0,1 | mittel bis stark |
| 4,32 ± 0,1 | stark |
| 4,16 ± 0,1 | schwach |
| 3,81 ± 0,1 | mittel bis stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,86 ± 0,1 | stark bis sehr stark |
| 2,80 ± 0,1 | schwach bis mittel |

Hierbei bedeutet $I_o$ die Intensität des stärksten Signals.

Für die Angabe der Intensitäten in Tabelle 1 gilt:

| relative Intensität | 100 $I/I_o$ |
|---|---|
| sehr stark | 80 - 100 |
| stark | 50 - 80 |
| mittel | 20 - 50 |
| schwach | 0 - 20 |

Für das Verhältnis Silicium zu Titan gilt im allgemeinen:

$$\frac{SiO_2}{SiO_2 + TiO_2} = 0,4 - 0,99,$$

vorzugsweise

$$\frac{SiO_2}{SiO_2 + TiO_2} = 0,7 - 0,99,$$

ausgedrückt in Molverhältnissen der Oxide.

Die erfindungsgemäßen neuen Zeolithe besitzen eine den Zeolithen T (US-PS 2 950 952) bzw. ZSM-34 (DE-OS 2 749 024) ähnliche Struktur, unterscheiden sich jedoch von diesen in der Zusammensetzung, insbesondere durch den Titangehalt.

Von den Titanosilicaten gemäß US-PS 3 329 481 und DE-OS 3 047 798 unterscheiden sich die erfindungsgemäßen titanhaltigen Zeolithe durch die Struktur.

Die erfindungsgemäßen Zeolithe lassen sich herstellen, indem man Titanverbindungen mit Aluminium-, Silicium-, Natrium-, Kalium-, Cholinverbindungen und Wasser mischt und in einem geschlossenen Gefäß erhitzt. Diesem Gemisch können darüberhinaus vor dem Erhitzen Impfkristalle zugesetzt werden.

Die Ausgangsverbindungen werden im allgemeinen in folgendem Verhältnis eingesetzt, ausgedrückt in Molverhältnissen der Oxide:

$$(SiO_2 + TiO_2) : (0,02 - 0,30)Al_2O_3 : (0,05 - 0,70)Na_2O : (0,02 - 0,30)K_2O : (0,02 - 0,5)R_2O : (10 - 90)H_2O,$$

vorzugsweise im Verhältnis

$(SiO_2 + TiO_2) : (0,02 - 0,18)Al_2O_3 : (0,10 - 0,60)Na_2O : (0,04 - 0,20)K_2O : (0,10 - 0,40)R_2O : (10 - 40)H_2O$,

wobei R gleich Cholin ist.

Für das Verhältnis Silicium zu Titan im Gemisch der Ausgangsverbindungen gilt im allgemeinen:

$$\frac{SiO_2}{SiO_2 + TiO_2} = 0,4 - 0,99,$$

vorzugsweise

$$\frac{SiO_2}{SiO_2 + TiO_2} = 0,6 - 0,99,$$

ausgedrückt in Molverhältnissen der Oxide.

Als Verbindungen können beispielsweise eingesetzt werden:

Kieselsäuregel, Kaliumsilicat, Natriumsilicat, Aluminiumhydroxid, Aluminiumsulfat, Natriumaluminat, Kaliumaluminat, Aluminiumhalogenide, Aluminiummetahydroxid, Titanhalogenide, Titansulfat, Titanoxidsulfat, Natriumtitanat, Kaliumtitanat, Titandioxid, Natriumhydroxid, Natriumsulfat, Natriumhalogenide, Kaliumhydroxid,Kaliumsulfat, Kaliumhalogenide, Cholinhydroxid, Cholinchlorid. Aber auch andere Silicium-, Aluminium-, Titan-, Kalium-, Natrium- und Cholinverbindungen eignen sich für die Herstellung der erfindungsgemäßen Zeolithe.

Das Gemisch der jeweils gewählten Verbindungen mit Wasser wird im allgemeinen 48 bis 2000 Stunden, vorzugsweise 48 bis 1000 Stunden lang, auf eine Temperatur zwischen 80 und 160°C, vorzugsweise zwischen 90 und 150°C, in einem geschlossenen Gefäß erhitzt.

Die gebildeten Zeolithe werden in üblicher Weise, z.B. durch Filtration, isoliert, gewaschen und getrocknet. Sie können nach bekannten Methoden in die katalytisch aktiven Formen überführt werden, z.B. durch Kalzinierung und/oder Ionenaustausch (D.W. Breck, Zeolite Molecular Sieves, 1974).

Die erfindungsgemäßen Zeolithe zeichnen sich nach ihrer Überführung in die katalytisch aktiven Formen insbesondere aus durch eine hohe Selektivität und durch eine geringe Koksabscheidung bei der Umwandlung von Methanol in niedere Olefine. Es ist überraschend, daß man mit Hilfe der angegebenen Methode überhaupt Zeolithe mit den erfindungsgemäßen Merkmalen erhält.

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-$\alpha$-Strahlung verwandt.

0094024

Beispiel 1

1,5 g Natriumaluminat (54 Gew.-% $Al_2O_3$, 41 Gew.-% $Na_2O$), 7,7 g Natriumhydroxid, 1,6 g Kaliumhydroxid und 12,7 g Cholinchlorid werden in 43 g Wasser gelöst. In diese Lösung werden unter intensivem Rühren zuerst 4,1 g Titantetrachlorid und dann 29,4 g 40 Gew.-%iges kolloidales Kieselgel eingebracht. Die entstandene Mischung wird homogenisiert und 8 Tage im geschlossenen Gefäß auf 150°C erhitzt. Das entstandene Produkt wird abfiltriert, mit Wasser gewaschen und bei 120°C getrocknet.

Das Produkt enthält Silicium, Aluminium und Titan in folgenden Anteilen, ausgedrückt in Molverhältnissen von Oxiden:

$$SiO_2 : 0{,}064\ Al_2O_3 : 0{,}175\ TiO_2$$

Das Ergebnis der Röntgenbeugungsanalyse ist in der Tabelle 2 wiedergegeben.

Tabelle 2

| Netzebenenabstände d/¯Å_7 | relative Intensität 100 $I/I_o$ |
|---|---|
| 11,45 | 100 |
| 9,16 | 3 |
| 7,56 | 14 |
| 6,62 | 41 |
| 6,33 | 15 |
| 5,74 | 18 |
| 5,34 | 3 |
| 4,56 | 42 |
| 4,33 | 51 |
| 4,14 | 6 |
| 3,80 | 44 |
| 3,76 | 89 |

Fortsetzung Tabelle 2

| Netzebenenabstände d/Å/ | relative Intensität 100 $I/I_o$ |
|---|---|
| 3,58 | 74 |
| 3,31 | 35 |
| 3,16 | 32 |
| 2,92 | 8 |
| 2,86 | 83 |
| 2,80 | 5 |
| 2,67 | 9 |
| 2,48 | 7 |

Beispiel 2

5,0 g Natriumaluminat, 19,5 g Natriumhydroxid, 4,4 g Kaliumhydroxid und 42 g Cholinchlorid werden in 135 g Wasser gelöst. In diese Lösung werden unter intensivem Rühren zuerst 102 g 40 Gew.-%iges kolloidales Kieselgel und dann 6,8 g Titantetrachlorid eingebracht. Die entstandene Mischung wird 30 Tage im geschlossenen Gefäß auf 105°C erhitzt. Das entstandene Produkt wird abfiltriert, mit Wasser gewaschen und bei 120°C getrocknet.

Das Produkt enthält Silicium, Aluminium und Titan in folgenden Anteilen, ausgedrückt in Molverhältnissen von Oxiden:

$$SiO_2 : 0,072\ Al_2O_3 : 0,103\ TiO_2.$$

Die Röntgendaten entsprechen den in Tabelle 1 angegebenen.

PATENTANSPRÜCHE

1. Titanhaltige Zeolithe, dadurch gekennzeichnet, daß sie

a) außer Natrium, Kalium und Cholin die Elemente Silicium, Aluminium und Titan in folgendem Verhältnis enthalten, ausgedrückt in Molverhältnissen von Oxiden:

$$(SiO_2 + TiO_2) : (0,02 - 0,30) Al_2O_3$$

und

b) im Röntgenbeugungsdiagramm die folgenden charakteristischen Signale aufweisen:

| Netzebenenabstände d [Å] | relative Intensität $I/I_o$ |
|---|---|
| 11,4 ± 0,3 | stark bis sehr stark |
| 9,2 ± 0,2 | schwach |
| 7,6 ± 0,2 | schwach bis mittel |
| 6,6 ± 0,1 | mittel bis stark |
| 5,7 ± 0,1 | schwach bis mittel |
| 5,35 ± 0,1 | schwach |
| 4,56 ± 0,1 | mittel bis stark |
| 4,32 ± 0,1 | stark |
| 4,16 ± 0,1 | schwach |
| 3,81 ± 0,1 | mittel bis stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,86 ± 0,1 | stark bis sehr stark |
| 2,80 ± 0,1 | schwach bis mittel |

wobei $I_o$ die Intensität des stärksten Signals bedeutet.

2. Titanhaltige Zeolithe nach Anspruch 1, dadurch gekennzeichnet, daß für das Verhältnis Silicium zu Titan gilt:

$$\frac{SiO_2}{SiO_2 + TiO_2} = 0,4 - 0,99,$$

ausgedrückt in Molverhältnissen der Oxide.

3. Titanhaltige Zeolithe nach Anspruch 1, dadurch gekennzeichnet, daß für das Verhältnis Silicium zu Titan gilt:

$$\frac{SiO_2}{SiO_2 + TiO_2} = 0,7 - 0,99,$$

ausgedrückt in Molverhältnissen der Oxide.

4. Verfahren zur Herstellung von titanhaltigen Zeolithen nach einem der Ansprüche 1 bis 3, dadurch gekennzeichnet, daß man eine Mischung aus Silicium-, Titan-, Aluminium-, Natrium-, Kalium-, Cholinverbindungen und Wasser herstellt, die folgende Zusammensetzung hat, ausgedrückt in Molverhältnissen der Oxide:

$$(SiO_2 + TiO_2) : (0,02 - 0,30)Al_2O_3 : (0,05 - 0,70)Na_2O : (0,02 - 0,30)\ K_2O : (0,02 - 0,5)R_2O : (10-90)H_2O,$$

wobei R gleich Cholin ist, und diese Mischung in einem geschlossenen Gefäß erhitzt.

5. Verfahren nach Anspruch 4, dadurch gekennzeichnet, daß die zu erhitzende Mischung folgende Zusammensetzung hat, ausgedrückt in Molverhältnissen der Oxide:

$(SiO_2 + TiO_2) : (0,02 - 0,18) Al_2O_3 : (0,10-0,60) Na_2O : (0,04 - 0,20) K_2O : (0,10 - 0,40) R_2O : (10-40) H_2O$,

wobei R gleich Cholin ist.

6. Verfahren nach Anspruch 4 oder 5, dadurch gekennzeichnet, daß für das Verhältnis Silicium zu Titan im Gemisch der Ausgangsverbindungen gilt:

$$\frac{SiO_2}{SiO_2 + TiO_2} = 0,4 - 0,99,$$

ausgedrückt in Molverhältnissen der Oxide.

7. Verfahren nach einem der Ansprüche 4 oder 5, dadurch gekennzeichnet, daß für das Verhältnis Silicium zu Titan im Gemisch der Ausgangsverbindungen gilt:

$$\frac{SiO_2}{SiO_2 + TiO_2} = 0,6 - 0,99,$$

ausgedrückt in Molverhältnissen der Oxide.

8. Verwendung von titanhaltigen Zeolithen nach einem der Ansprüche 1 bis 3 als Katalysatoren bei der Herstellung von $C_2$ bis $C_4$-Olefinen aus Methanol.

Europäisches Patentamt

# EUROPÄISCHER RECHERCHENBERICHT

0094024

Nummer der Anmeldung

EP 83 10 4387

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| P,X | EP-A-0 077 522 (HOECHST) * Ansprüche 1, 5 * | 1,4 | C 01 B 33/28<br>B 01 J 29/28 |
| D,Y | DE-A-2 749 024 (MOBIL OIL) * Ansprüche 5, 6 * | 1,4 | |
| D,Y | US-A-3 329 481 (D.A. YOUNG) * Anspruch 1 * | 1,4 | |
| A | EP-A-0 027 736 (NATIONAL DEVELOPMENT CORP.) | | |
| A | DE-A-1 667 727 (P. SPENCE & SONS) * Seiten 29-30 * | | |
| A | US-A-4 022 714 (C.H. ELLIOTT) | | RECHERCHIERTE SACHGEBIETE (Int. Cl. 3) |
| D,A | US-A-2 950 952 (D.W. BRECK et al.) | | C 01 B 33/00<br>B 01 J 29/00 |

Der vorliegende Recherchenbericht wurde für alle Patentansprüche erstellt.

| Recherchenort | Abschlußdatum der Recherche | Prüfer |
|---|---|---|
| BERLIN | 12-08-1983 | KESTEN W |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82